# EUROPEAN PATENT SPECIFICATION

(11) **EP 4 208 222 B1**
(45) Date of publication and mention of the grant of the patent: **06.05.2026**
(21) Application number: 21769745.7
(22) Date of filing: 31.08.2021
(51) Int. Cl.: A61M 5/315, A61M 11/00, A61M 11/08, A61M 15/08, B05B 11/02

(54) **DRUG SYRINGES WITH A MECHANICAL STOP FOR A SECOND DOSE**
ARZNEIMITTELSPRITZEN MIT MECHANISCHEM ANSCHLAG FÜR EINE ZWEITE DOSIS
SERINGUES DE MÉDICAMENT DOTÉES D'UNE BUTÉE MÉCANIQUE POUR UNE SECONDE DOSE

(30) Priority: 01.09.2020 US 202063073032 P
(43) Date of publication of application: 12.07.2023
(73) Proprietor: Janssen Pharmaceutica, N.V., 2340 Beerse (BE)
(72) Inventor: HUBERT, Emma, Louise, Milpitas, California 95035 (US); SCRIMGEOUR, Ian, Dunbar EH42 ILT (GB); RAMOS, David, Orange Park, Florida 32065 (US); VESOLE, Steven, M., Milpitas, California 95035 (US); KAPIL, Monica A., San Jose, California 95127 (US); GEARY, William C., Boston, Massachusetts 02111 (US); POPLI, Shagun, Milpitas, California 95035 (US); CANNAMELA, Michael, New York 10001 (US); NGUYEN, Jimmy, Vinh, Hoang, Milpitas, California 95035 (US); YAN, Hong, Milpitas, California 95035 (US); PÉREZ, Dolores, Easton, Pennsylvania 18045 (US)
(74) Representative: Carpmaels & Ransford LLP
(86) International application number: PCT/EP2021/074039
(87) International publication number: WO 2022/049080

(56) References cited:
- EP-B1- 0 526 824
- WO-A1-2013/145789
- GB-A- 758 769
- JP-B2- 5 888 234
- KR-B1- 102 066 186
- US-A- 2 764 981
- US-A- 2 856 925
- US-A- 2 869 541
- US-A- 4 962 868
- US-A1- 2005 004 530
- US-A1- 2010 286 513
- US-A1- 2020 030 552

## Description

### FIELD

The present disclosure relates generally to drug syringes with a mechanical stop for a second dose and drug products utilizing the same.

### BACKGROUND

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. These devices require a series of operational steps that the user needs to properly carry out to effect optimal drug delivery through self-administration.

Accordingly, there remains a need for improved nasal drug delivery devices.

In JP5888234B2, there is described an administration device capable of discharging a prescribed, equal amount of medicine a number of times by spraying.

In EP0526824B1, there is described a syringe nasal sprayer that includes an elongate barrel having an open proximal end, a chamber for retaining fluid and a tip portion extending from a distal end of the barrel having a passageway therethrough communicating with the chamber.

In US2764981A, there is described dispensing devices, and more particularly an improved multiple dosage syringe that automatically indicates when each of a number of doses of predetermined volume has been dispensed therefrom.

In US2856925A, there is described dispensing devices, and more particularly an improved multiple dosage syringe that automatically indicates when each of a number of doses of predetermined volume has been dispensed therefrom.

In KR102066186B1, there is described a syringe for mixing and ejecting an active pharmaceutical ingredient.

In US4962868A, there is described an apparatus for dispensing a controlled dose of a liquid fluid such as a liquid medication that includes a dispenser head which is snugly fitted over the needle-shaped end of a syringe filled with the liquid pharmaceutical and including a piston in fully retracted or drawn position.

In US2010286513A1, there is described a syringe assembly for sequentially injecting multiple medical fluids.

In GB758769A, there is described a holder for a syringe ampoule that holds the ampoule at one end only and provides a plunger rod for the expulsion of medicament.

In US2869541A, there is described dispensing devices, and more particularly an improved multiple dosage syringe that automatically indicates when each of a number of doses of predetermined volume has been dispensed therefrom.

In US2005004530A1, there is described a syringe that may include various forms of a releasable stop adapted to selectively limit the travel of the syringe plunger within the syringe barrel such that a first amount of therapeutic substance may be discharged from the syringe while a residual supply of the therapeutic substance remains in the syringe.

In WO2013145789A1, there is described a metered-dose syringe type spray device in which multiple stages of pushing are possible.

In US2020030552A1, there is described a syringe type ejection device capable of reducing an amount of residual liquid is provided.

### SUMMARY

In general, drug syringes with a mechanical stop for a second dose, drug products utilizing the same, and methods of using drug syringes with a mechanical stop for a second dose are provided.
The invention is defined by appended claim 1. Further embodiments are defined by the dependent claims.

### BRIEF DESCRIPTION OF DRAWINGS

The present invention is described by way of reference to Figure 1 and Figures 18 to 21. Further examples of other embodiments not according to the invention can be found in the remaining figures as follows:
FIG. 1 is a block diagram of one embodiment of a drug delivery device;
FIG. 2 is a perspective view of one embodiment of the drug delivery device of FIG. 1;
FIG. 3 is a cross-sectional view of a portion of the drug delivery device of FIG. 2;
FIG. 4 is a perspective view of another embodiment of the drug delivery device of FIG. 1;
FIG. 5 is a perspective view of yet another embodiment of the drug delivery device of FIG. 1;
FIG. 6 is a perspective view of another embodiment of a stop member of the drug delivery device of FIG. 1;
FIG. 7 is another view of the stop member of FIG. 6;
FIG. 8 is yet another view of the stop member of FIG. 6;
FIG. 9 is a side partially transparent view of another embodiment of the drug delivery device of FIG. 1;
FIG. 10 is a perspective view of the drug delivery device of FIG. 9;
FIG. 11 is a side view of a portion of the drug delivery device of FIG. 9;
FIG. 12 is a side view of a portion of the drug delivery device of FIG. 11 after a first drug dose has been delivered;
FIG. 13 is a side view of a portion of the drug delivery device of FIG. 12 after a second drug dose has been delivered;
FIG. 14 is a side partially transparent view of yet another embodiment of the drug delivery device of Fig. 1.
FIG. 15 is a side view of a portion of the drug delivery device of FIG. 14;
FIG. 16 is a top view of a body of the drug delivery device of FIG. 14;
FIG. 17 is a bottom view of the body of the drug delivery device of FIG. 14;
FIG. 18 is a side partially transparent view of the drug delivery device according to the invention;
FIG. 19 is a side cross-sectional view of a portion of the drug delivery device of FIG. 18;
FIG. 20 is a side cross-sectional view of another embodiment of a depressible pin of the drug delivery device of FIG. 18;
FIG. 21 is a perspective view of the drug delivery device of FIG. 18 and one embodiment of an accessory;
FIG. 22 is a perspective partially transparent view of another embodiment of the drug delivery device of FIG. 1;
FIG. 23 is a side cross-sectional view of a portion of the drug delivery device of FIG. 22;
FIG. 24 is an exploded side perspective partially transparent view of another embodiment of the drug delivery device of FIG. 1;
FIG. 25 is a side partially transparent view of still another embodiment of the drug delivery device of FIG. 1;
FIG. 26 is a side view of a portion of the drug delivery device of FIG. 25;
FIG. 27 is a side cross-sectional view of another embodiment of the drug delivery device of FIG. 1;
FIG. 28 is a side cross-sectional view of the drug delivery device of FIG. 27 with a first drug dose being delivered; and
FIG. 29 is a side cross-sectional view of the drug delivery device of FIG. 28 with a second drug dose being delivered.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary drug syringes with a mechanical stop for a second dose, drug products utilizing the same, and methods of using drug syringes with a mechanical stop for a second dose are provided. In general, a nasal drug delivery device is configured to dispense therefrom first and second doses of a drug therefrom into a nose. The drug delivery device includes a mechanical stop configured to provide a pause between the delivery of the first and second doses. During the pause, a user of the drug delivery device can move the drug delivery device from one nostril, into which the first dose was sprayed, to another nostril, into which the second dose can be sprayed. The mechanical stop is configured to hold the drug delivery device in a static delivery state such that the user can choose a time to begin delivery of the second dose, e.g., when to actuate the drug delivery device for a second time.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

A drug delivery device configured to expel a drug into a nose of a patient and to drive delivery of the drug using a propellant can have a variety of configurations. In general, the drug delivery device is configured to deliver a drug to a patient, where the drug is provided in a defined dosage form within the drug delivery device.

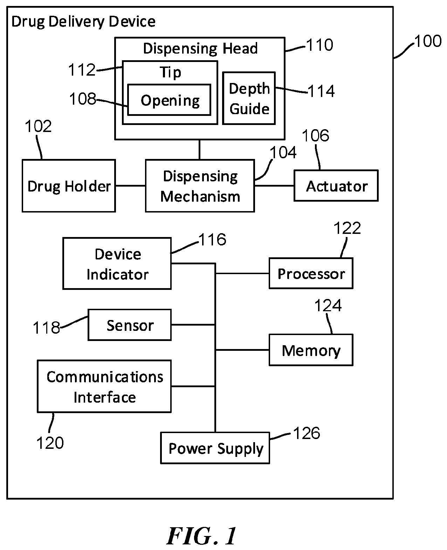

FIG. 1 illustrates one embodiment of a drug delivery device 100 configured to expel a drug into a nose of a patient. As will be appreciated by a person skilled in the art, the drug delivery device 100 can include different features in different embodiments depending upon various requirements, such as the type of drug, typical dosage(s) of the drug, safety requirements in various jurisdictions, whether the device is powered, etc.

The drug delivery device 100 includes a drug holder 102 configured to contain a drug therein for delivery from the device 100 to a patient. The drug holder 102 can have a variety of configurations, such as a cartridge, a vial, a blow-fill-seal (BFS) capsule, a blister pack, etc.

The drug delivery device 100 also includes a dispensing mechanism 104 that is operatively coupled to the drug holder 102 and configured to drive the drug out of device 100 from the drug holder 102. The dispensing mechanism 104 can have a variety of configurations. For example, the dispensing mechanism 104 can include a plunger configured to push the drug out of the drug holder 102. For another example, the dispensing mechanism 104 can include a piston, pin, and/or a needle configured to pierce through or puncture a seal member of the drug holder 102 in embodiments in which the drug holder 102 includes a pierceable or puncturable seal member.

The drug delivery device 100 also includes an actuator 106 configured to be actuated by a user to cause the dispensing mechanism 104 to begin delivering a dose of the drug through an opening or nozzle 108 in the drug delivery device 100. In an exemplary embodiment, the drug delivery device 100 is configured to be self-administered such that the user who actuates the actuator 106 is the patient receiving the drug from the drug delivery device 100, although another person can actuate the device 100 for delivery into another person. The actuator 106 can have a variety of configurations, as discussed further below. For example, the actuator 106 can include a plunger. For another example, the actuator 106 can include a pressable button. For still another example, the actuator 106 can include a movable switch. For yet another example, the actuator 106 can include a squeezable body of the drug holder 102.

The opening 108 through which the drug exits the drug delivery device 100 is formed in a dispensing head 110 of the drug delivery device 100 in a tip 112 of the dispensing head 110. The tip 112 is configured to be inserted into a nostril of a patient. In an exemplary embodiment, the tip 112 is configured to be inserted into a first nostril of the patient during a first stage of operation of the drug delivery device 100 and into a second nostril of the patient during a second stage of operation of the drug delivery device 100. The first and second stages of operation involve two separate actuations of the actuator 106, a first actuation corresponding to a first dose of the drug being delivered and a second actuation corresponding to a second dose of the drug being delivered. The dispensing head 110 includes a depth guide 114 configured to contact skin of the patient, e.g., between the patient's first and second nostrils, such that a longitudinal axis of the dispensing head 110 is substantially aligned with a longitudinal axis of the nostril in which the tip 112 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, the dispensing head 110 has a tapered shape in which the dispensing head 110 has a smaller diameter at its distal end than at its proximal end where the opening 108 is located. The opening 108 having a relatively small diameter facilitates spray of the drug out of the opening 108, as will be appreciated by a person skilled in the art. A spray chamber through which the drug is configured to pass before exiting the opening 108 is located within a proximal portion of the tapered dispensing head 110, distal to the opening 108. When the drug passes through the spray chamber at speed, the spray chamber facilitates production of a fine mist that exits through the opening 108 with a consistent spray pattern.

In some embodiments, the dispensing head 110 can include two tips 112 each having an opening 108 therein such that the drug delivery device 100 is configured to simultaneously deliver doses of drug into two nostrils in response to a single actuation of the actuator 106.

The drug delivery device 100 also includes a device indicator 116 configured to present information to a user about a status of the drug delivery device 100 and/or the drug contained in the drug holder 102. The device indicator 116 can be a visual indicator, such as a display screen, one or more lights, one or more colored and/or numbered markings, etc. Alternatively or in addition, the device indicator 116 can be an audio indicator configured to provide sound.

The drug delivery device 100 also includes a sensor 118 configured to sense information relating to the drug delivery device 100 and/or the drug contained in the drug holder 102. Examples of information that the sensor 118 can sense include environmental conditions (e.g., temperature, humidity, geographic location, time, etc.). The drug delivery device 100 can also include a communications interface 120 configured to communicate externally data which has been gathered by the sensor 118 about the drug delivery device 100 and/or the drug contained in the drug holder 102, which may facilitate analysis regarding the patient's treatment, patient compliance, use of the drug delivery device 100, etc.

In embodiments in which the drug delivery device 100 includes one or more electrical components, e.g., the device indicator 116 (which in some embodiments can be powered while in other embodiments not be powered), the sensor 118, the communications interface 120, a processor 122, a memory 124, etc., the drug delivery device 100 includes a power supply 126 configured to deliver electrical power to the one or more electrical components of the drug delivery device 100. The power supply 126 can be a source of power which is integral to drug delivery device 100 and/or can be a mechanism configured to connect the drug delivery device 100 to an external source of power. The processor 122 is configured to receive gathered data from the sensor 118 and to cause the data to be stored in the memory 124, to be indicated on the device indicator 110, and/or and to be communicated externally via the communications interface 120. The memory 124 is configured to store instructions that are configured to be executed by the processor 122 for the processor 122 to process information regarding the various electrical components with which the processor 122 is in communication.

As mentioned above, the drug delivery device 100 can include different features in different embodiments depending upon various requirements. For example, the drug delivery device 100 can omit any one or more of the depth guide 114, the device indicator 116, the sensor 118, the communications interface 120, the processor 122, the memory 124, and the power supply 126.

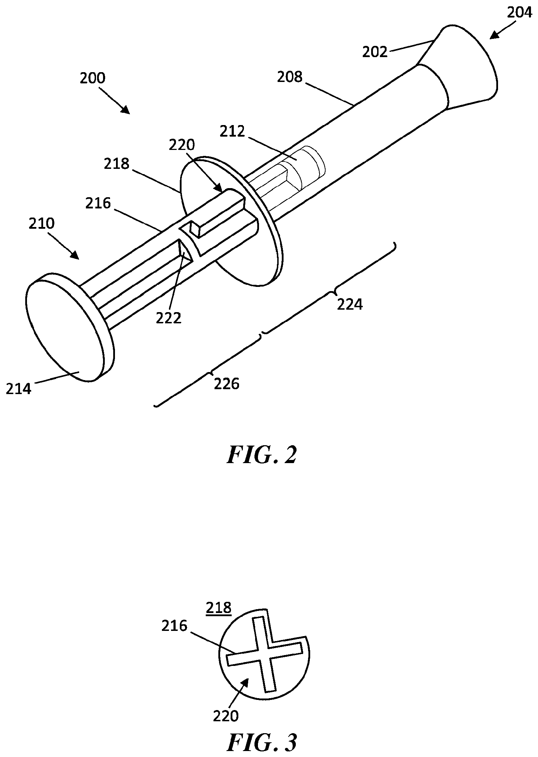

FIG. 2 illustrates an exemplary embodiment of the drug delivery device 100 of FIG. 1. FIG. 2 illustrates a drug delivery device 200 configured to expel a drug into a nose of a patient in two stages of operation. In a first stage of operation a first dose of the drug is delivered, and in a second stage of operation a second dose of the drug is delivered.

The drug delivery device 200 is generally configured and used similar to that discussed above regarding FIG. 1 and includes a dispensing head 202 that includes an opening 204 therein. The dispensing head 202 in this illustrated embodiment is not configured to be disposed in a nose but instead to abut a bottom surface of a nose under a nostril to deliver drug into the nostril. The drug is contained in a chamber defined by a body 208 of the drug delivery device. The drug is configured to be delivered from the drug holder and into a first nostril during a first stage of operation of the drug delivery device 200 and into a second nostril during a second stage of operation of the drug delivery device 200. The drug in this illustrated embodiment is configured to be self-administered such that the user who actuates the drug delivery device is the person receiving the drug from the drug delivery device 200, although another person can actuate the device 200 for delivery into another person.

An actuator of the drug delivery device 200 includes a plunger 210 that is configured to be pushed distally toward the dispensing head 204 to cause the drug to be delivered from the drug delivery device 200. The plunger 210 is operatively engaged with a stopper 212 located proximal to the drug contained in the chamber . The plunger's distal movement is configured to cause corresponding distal movement of the stopper 212 to push drug from the chamber and out of the opening 204. The plunger 210 includes a proximal flange 214 configured to be pushed by a user, e.g., with a thumb thereof, to actuate the drug delivery device 200 and cause the plunger 210 to move distally relative to the body 208 and the dispensing head 204 to cause the drug delivery. The plunger 210 also includes an elongate shaft 216 that extends distally from the proximal flange 214.

The plunger 210 and the body 208 are configured to cooperate with one another to allow for two doses to be sequentially delivered from the drug delivery device 200. **In** other words, the plunger 210 and the body 208 are configured to cooperate with one another to provide for the first and second stages of operation. The elongate shaft 216 of the plunger 210 is configured to cooperate with a proximal flange 218 of the body 208 to allow for the two doses to be sequentially delivered from the drug delivery device 200.

As also shown in FIG. 3, the body's proximal flange 218 includes a hole 220 therein in which the elongate shaft 216 is configured to slide in the first and second stages of operation. The hole 220 has a shape that cooperates with a cross-sectional shape of the elongate shaft 214 to allow for the two doses to be sequentially delivered from the drug delivery device 200. The hole 220 has a circular sector, e.g., a "Pac-Man" shape, in this illustrated embodiment. The elongate shaft 216 an "X" cross-sectional shape along its longitudinal length. However, a stop member 222 is located on the elongate shaft 216 in an intermediate area thereof. A first longitudinal length 224 of the elongate shaft 216 distal to the stop member 222 defines a first distance that the plunger 210 moves distally in the first stage of operation, and a second longitudinal length 226 of the elongate shaft 216 proximal to the stop member 222 defines a distance that the plunger 210 moves distally in the second stage of operation. The stop member 222, e.g., a distal surface thereof, is configured to abut the body's proximal flange 218, e.g., a proximal surface thereof, to stop distal movement of the plunger 210 and thus end the first stage of operation. The plunger 210 can then be rotated relative to the body 208 so the stop member 222 no longer impedes distal movement of the plunger 210. An amount of the rotation will depend on a size of the hole 220 and stop member 222. In this illustrated embodiment, the plunger 210 is configured to be rotated about ninety degrees to cause the stop member 222 to be aligned with the hole 220 instead of the solid flange 218 surface. A person skilled in the art will appreciate that the rotation may not be precisely ninety degrees but nevertheless be considered to be substantially ninety degrees due to any number of factors, such as manufacturing tolerances and sensitivity of measurement equipment. After the plunger 210 has been rotated, the plunger 210 can be actuated again, e.g., pushed distally again, so the stop member 222 and the second longitudinal length 226 of the elongate shaft 216 passes through the hole 220 in the second stage of operation.

In some embodiments, the drug delivery device 200 includes a flexible clip feature configure to provide a trigger force. The flexible clip feature can be attached to the elongate shaft 216 such that in response to actuation of the plunge 210, e.g., to pushing on the plunger's proximal flange 214, the flexible clip feature flexes and becomes disengaged to urge the plunger 210 distally, e.g., under a spring-biased force. The plunger 210 may therefore push the stopper 212 at a higher speed, which may cause the drug to be sprayed in a finer mist out of the opening 204.

The drug delivery device 200 in this illustrated embodiment is not powered, e.g., does not include any electrical components such as a processor, a sensor, a memory, a communications interface, etc. The drug delivery device 200 thus does not need to include a power supply though a power supply could be included, e.g., for connectivity, for powering a light source of the drug delivery device, etc.

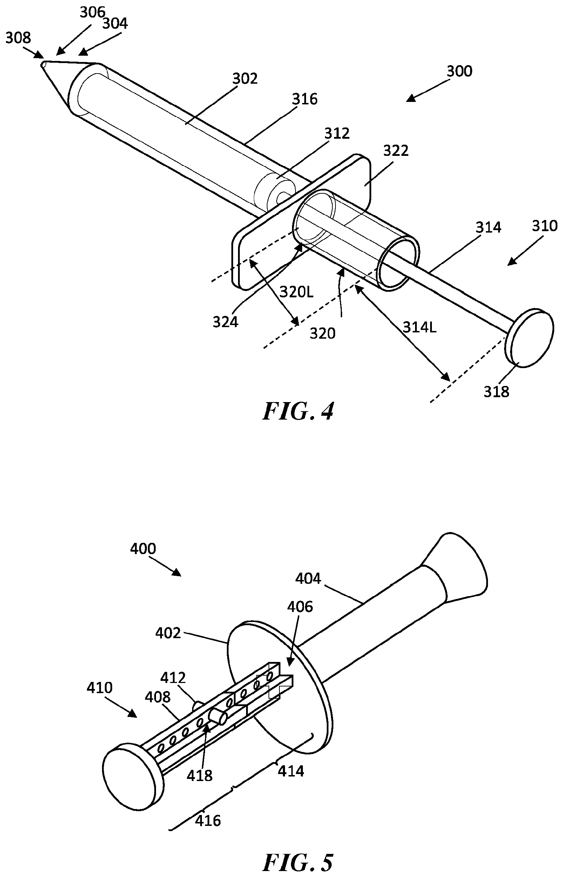

FIG. 4 illustrates another exemplary embodiment of the drug delivery device 100 of FIG. 1. FIG. 4 illustrates a drug delivery device 300 configured to expel a drug into a nose of a patient in two stages of operation. In a first stage of operation a first dose of the drug is delivered, and in a second stage of operation a second dose of the drug is delivered. The drug delivery device 300 is generally configured and used similar to that discussed above regarding FIG. 1 and includes a drug holder 302 and a dispensing head 304 that includes a tip 306 and an opening 308. The tip 306 is configured to be inserted into a first nostril during a first stage of operation of the drug delivery device 300 and into a second nostril during a second stage of operation of the drug delivery device 300. The drug in this illustrated embodiment is configured to be self-administered such that the user who actuates the drug delivery device 300 is the person receiving the drug from the drug delivery device 300, although another person can actuate the device 300 for delivery into another person.

In this illustrated embodiment, an actuator of the drug delivery device 300 includes a plunger 310 that is configured to be pushed distally toward the dispensing head 304 to cause the drug to be delivered from the drug delivery device 300. The plunger 310 is operatively engaged with a stopper 312 located proximal to the drug in the drug holder 302. The plunger's distal movement is configured to cause corresponding distal movement of the stopper 312 to push drug from the drug holder and out of the opening 308. The plunger 310 includes a proximal flange 314 configured to be pushed by a user, e.g., with a thumb thereof, to actuate the drug delivery device 300 and cause the plunger 310 to move distally relative to a body 316 of the drug delivery device 300 and relative to the dispensing head 304 to cause the drug delivery. The plunger 310 also includes an elongate shaft 318 that extends distally from the proximal flange 314.

The drug delivery device 300 includes a removable tab 320 configured to facilitate operation of the drug delivery device 300 with first and second stages of operation. The removable tab 320 is removably coupled to the body 316, e.g., to a proximal flange 322 of the body 316. The removable tab 320 can be removably coupled to the body 316 in any of a variety of ways. For example, as in this illustrated embodiment, the removable tab 320 can be removably coupled to the body 316 at a perforation 324 at which the removable tab 320 is configured to be torn from the body 316. For another example, the removable tab 320 can be removably coupled to the body 316 using a releasable adhesive similar to Post-it^{®} note adhesive. For yet another example, the removable tab 320 can be removably coupled to the body 316 using Velcro^{®}. For still another example, the removable tab 320 can be removably coupled to the body 316 by having a distal portion thereof seated in a slot formed in the body's proximal flange 322.

The plunger 310 and the removable tab 320 are configured to cooperate with one another to allow for two doses to be sequentially delivered from the drug delivery device 300. **In** other words, the plunger 310 and the removable tab 320 are configured to cooperate with one another to provide for the first and second stages of operation.

The removable tab 320 extends proximally from body 306. The plunger 310 also extends proximally from the body 316. The removable tab 320 has a longitudinal length 320L extending proximally from the body 306 that is less than a longitudinal length 314L of the plunger's elongate shaft 314 that extends proximally from the body 316 to the plunger's proximal flange 318 with the drug delivery device 300 in an initial configuration, which is shown in FIG. 4. The longitudinal length 314L of the plunger's elongate shaft 314 that extends proximally from the body 316 to the plunger's proximal flange 318 defines a first distance that the plunger 310 moves distally in the first stage of operation, and the longitudinal length 320L of the removable tab 320L that extends proximally from the body 306 defines a distance that the plunger 310 moves distally in the second stage of operation. The removable tab 320, e.g., a proximal surface thereof, is configured to abut the plunger's proximal flange 318, e.g., a distal surface thereof, to stop distal movement of the plunger 310 and thus end the first stage of operation. The removable tab 320 can then be removed from the body 306 so the removable tab 320 no longer impedes distal movement of the plunger 310. After the removable tab 320 has been removed, the plunger 310 can be actuated again, e.g., pushed distally again, to cause a drug dose delivery in the second stage of operation.

The drug delivery device 300 in this illustrated embodiment is not powered, e.g., does not include any electrical components such as a processor, a sensor, a memory, a communications interface, etc. The drug delivery device 300 thus does not need to include a power supply though a power supply could be included, e.g., for connectivity, for powering a light source of the drug delivery device, etc.

FIG. 5 illustrates another exemplary embodiment of the drug delivery device 100 of FIG. 1. FIG. 5 illustrates a drug delivery device 400 configured to expel a drug into a nose of a patient in two stages of operation. **In** a first stage of operation a first dose of the drug is delivered, and in a second stage of operation a second dose of the drug is delivered. The drug delivery device 400 is generally configured and used similar to that discussed above regarding the drug delivery device 200 of FIG. 2 except that the drug delivery device 400 of FIG. 5 includes a different stop member configuration.

In the illustrated embodiment of FIG. 5, a proximal flange 402 of the drug delivery device's body 404 includes a hole 406 therein in which an elongate shaft 408 of the drug delivery device's plunger 410 is configured to slide in the first and second stages of operation. The hole 406 has a shape that cooperates with a cross-sectional shape of the elongate shaft 408 to allow for the two doses to be sequentially delivered from the drug delivery device 400. The hole 406 has an "X" shape in this illustrated embodiment, and the elongate shaft 408 has a corresponding "X" cross-sectional shape along its longitudinal length. A stop member 412 is located on the elongate shaft 408 in an intermediate area thereof similar to the stop member 222 of FIG. 2. However, in this illustrated embodiment, the plunger 410 does not need to be rotated to remove the impediment of the stop member 412. Instead, the stop member 412 is releasably coupled to the plunger 410, e.g., to the elongate shaft 408 thereof, such that the stop member 412 is removable from the plunger 410. The stop member 412, e.g., a distal surface thereof, is configured to abut the body's proximal flange 402, e.g., a proximal surface thereof, to stop distal movement of the plunger 410 and thus end the first stage of operation after the plunger 410 has moved a first longitudinal distance 414. The stop member 412 can then be removed from the lounger 410 so the stop member 412 no longer impedes distal movement of the plunger 410. **In** this illustrated embodiment, the stop member 412 is in the form of a pin positioned in an opening 418 formed through the elongate shaft 408 of the plunger 410 that is configured to me removed from the plunger 410 by being manually slid out of the opening 418. After the stop member 412 has been removed, the plunger 410 can be actuated again, e.g., pushed distally again, so the elongate shaft 408 further passes through the hole 406 and the plunger 410 moves a second longitudinal distance 416 in the second stage of operation.

The lengths of the first and second longitudinal distances 414, 416 is defined by a total longitudinal length of the elongate shaft 408 and where the stop member 412 is located along the elongate shaft's total longitudinal length. In an exemplary embodiment, the first and second longitudinal distances 414, 416 are substantially equal such that the first and second drug doses are substantially equal. A person skilled in the art will appreciate that the values may not be precisely equal but nevertheless be considered to be substantially equal due to any number of factors, such as manufacturing tolerances and sensitivity of measurement equipment. In such embodiments, as illustrated in FIG. 5, the opening 418 in which the stop member 412 is located during use of the drug delivery device 400 until after the first drug delivery has occurred is located substantially at a midpoint of the elongate shaft's total longitudinal length. A person skilled in the art will appreciate that the opening 418 may not be precisely at the midpoint but nevertheless be considered to be substantially at the midpoint due to any number of factors, such as manufacturing tolerances and sensitivity of measurement equipment. In other embodiments, the first and second longitudinal distances 414, 416 are different from one another such that the first and second drug doses are different from one another. The elongate shaft 408 can, as in this illustrated embodiment, include a plurality of openings 418 along the elongate shaft's longitudinal length that are each configured to removably seat the stop member 412 therein. The opening 418 in which the stop member 412 is located will define the first and second longitudinal distances 414, 416 and thus the first and second drug dose amounts. Alternatively, only one opening 418 can be present such that the drug delivery device 400 is configured to deliver preset first and second drug dose amounts.

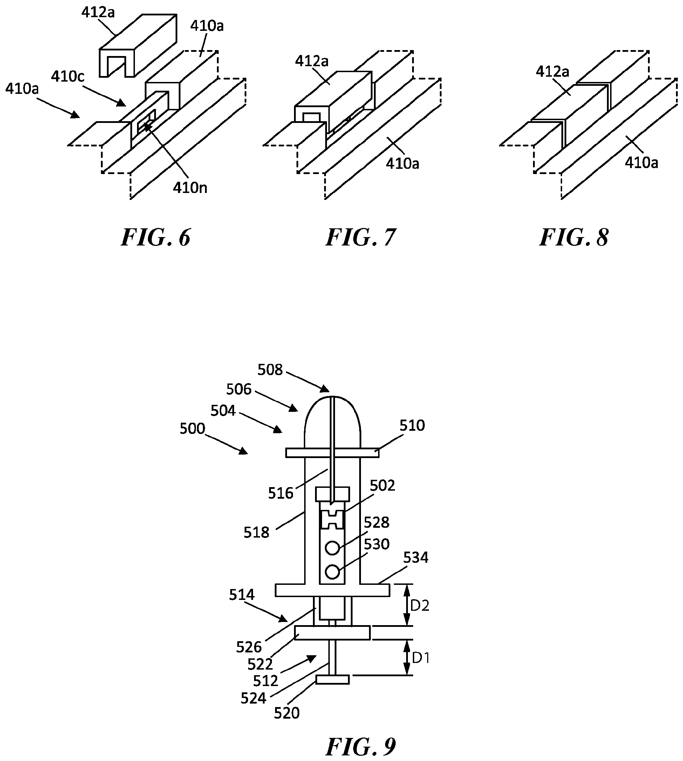

FIGS. 6 and 7 illustrates another embodiment of the stop member 412 and the plunger 410. In this illustrated embodiment, the stop member 412a includes a clip removably seated in a cut-out 410c formed in the plunger's elongate shaft 408a and clipped around a notch 410n therein. FIG. 6 illustrates the stop member 412a being coupled to the elongate shaft 408a. FIG. 7 illustrates the stop member 412a removably seated in the cut-out 410c. The stop member 412a has a size such that the stop member 412a protrudes radially outward beyond the elongate shaft 410a when removably coupled to the plunger 410a, as shown in FIG. 7. The stop member 412a is thus configured to abut the drug delivery device's body as discussed above with respect to the stop member 412. The stop member 412a can be removed from the plunger 410a by being pulled out of the cut-out 410c. The stop member 412a can include a spring-biased base that allows the stop member 412a to be so removed. Alternatively, instead of being removed from the plunger 410, the stop member 412a can be pushed farther into the cut-out 410c to no longer protrude radially outward beyond the elongate shaft 410a, as shown in FIG. 8. The stop member 412a can include an internal spring-biased member, similar to a carabiner, that allows the stop member 412a to be pushed down and clip around the notch 410n with the stop member 412a positioned farther down in the cut-out 410c.

The drug delivery device 400 in this illustrated embodiment is not powered, e.g., does not include any electrical components such as a processor, a sensor, a memory, a communications interface, etc. The drug delivery device 400 thus does not need to include a power supply though a power supply could be included, e.g., for connectivity, for powering a light source of the drug delivery device, etc.

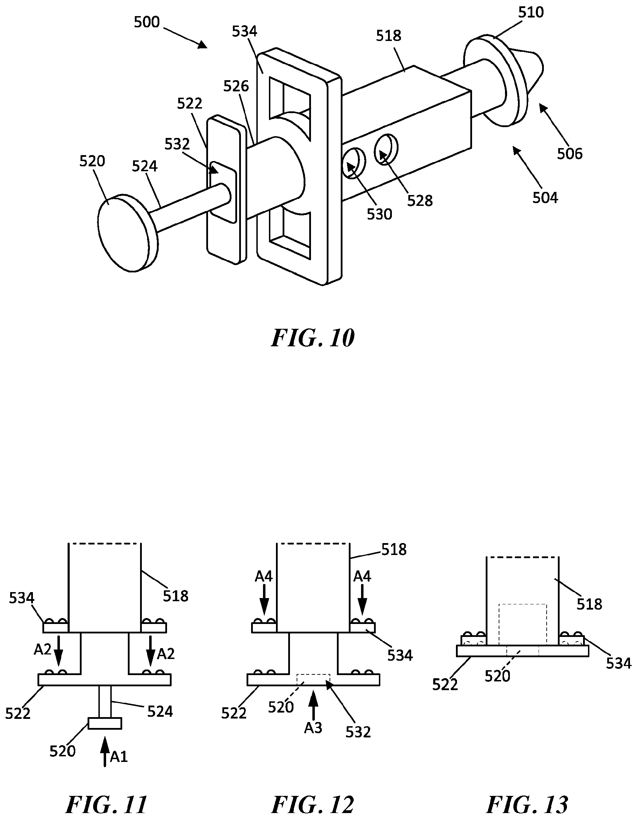

FIGS. 9 and 10 illustrate another exemplary embodiment of the drug delivery device 100 of FIG. 1. FIGS. 9 and 10 illustrate a drug delivery device 500 configured to expel a drug into a nose of a patient in two stages of operation. In a first stage of operation a first dose of the drug is delivered, and in a second stage of operation a second dose of the drug is delivered. The drug delivery device 500 is generally configured and used similar to that discussed above regarding FIG. 1 and includes a drug holder 502 and a dispensing head 504 that includes a tip 506, an opening 508, and a depth guide 510. The tip 506 is configured to inserted into a first nostril during a first stage of operation of the drug delivery device 500 and into a second nostril during a second stage of operation of the drug delivery device 500. The drug in this illustrated embodiment is configured to be self-administered such that the user who actuates the drug delivery device 500 is the person receiving the drug from the drug delivery device 500, although another person can actuate the device 500 for delivery into another person.

The drug holder 502 in this illustrated embodiment is a vial. The vial 502 includes a seal member at a proximal end thereof that is configured to provide a fluid tight seal such that the drug is contained in the vial 502 until the seal provided by the seal member is broken. The seal provided by the seal member can be broken in a variety of ways, such as by being pierced by a needle 516 of the drug delivery device 500, as discussed further below. The seal member can have a variety of configurations, as will be appreciated by a person skilled in the art, such as by being a pierceable polymer septum or a foil layer. The seal member can be protected from accidental puncturing or piercing before intended use with a removable protective member or stopper, such as a tamper evident (TE) seal, etc. In some embodiments, the seal member can be omitted and instead a removable protective member or stopper can be provided that is removed just prior to use of the vial 502.

In this illustrated embodiment, an actuator of the drug delivery device 500 includes a first plunger 512 configured to be pushed distally toward the dispensing head 504 to cause the drug to be delivered from the drug delivery device 500 in a first stage of operation. The actuator also includes a second plunger 514 configured to be pushed distally toward the dispensing head 504 to cause the drug to be delivered from the drug delivery device 500 in a second stage of operation. The first and second plungers 512, 514 are each operatively engaged with the drug holder 502. Each of the plungers' distal movement is configured to cause corresponding distal movement of the drug holder 502 such that drug is urged out of the drug holder 502, through the needle 516, and out of the opening 508. Each of the plungers 512, 514 includes a proximal flange 520, 522 configured to be pushed by a user, e.g., with a thumb thereof, to actuate the drug delivery device 500 and cause its associated plunger 512, 514 to move distally relative to a body 518 of the drug delivery device 500 and relative to the dispensing head 504 to cause the drug delivery. The plungers 512, 514 also each include an elongate shaft 524, 526 that extends distally from its associated proximal flange 520, 522.

The drug delivery device 500 also includes first and second viewing windows 528, 530 formed in a side wall of the body 518. The first and second viewing windows 528, 530 are configured to be clearly visible to a user of the drug delivery device 500 when the drug delivery device 500 is held by hand. The viewing windows 528, 530 are circular in this illustrated embodiment but can have another shape, e.g., square, triangular, ovular, etc. The drug delivery device 500 includes only two viewing windows 528, 530 in this illustrated embodiment but can have another number of viewing windows. For example, third and fourth viewing windows can be formed in a side wall on an opposite side of the body 518 that are configured and used similar to the first and second viewing windows 528, 530. The third and fourth viewing windows may facilitate a user's visualization of at least one pair of viewing windows, e.g., the first and second viewing windows 528, 530 or the third and fourth viewing windows, regardless of whether the user's right or left hand is holding the drug delivery device 500.

The viewing windows 528, 530 are configured to cooperate with a device indicator of the drug delivery device 500. The device indicator is configured to indicate to a user whether a first dose has been dispensed, e.g., whether the first stage of operation has occurred, and whether a second dose has been dispensed, e.g., whether the second stage of operation has occurred. The device indicator is configured to be visible through the viewing windows 528, 530 to indicate whether the first and second doses have been dispensed. In this way, the user can know a drug delivery status of the drug delivery device 500, including whether or not one or both of the first and doses have been dispensed. The drug holder 502 in this illustrated embodiment has an amount of drug disposed therein for two doses, so indicating that two doses have been delivered thus indicates that substantially no drug remains in the drug holder 502. Some drugs, such as esketamine, ketamine, and other controlled substances, may be required to have drug delivery therefrom verified per the drug's Risk Evaluation and Mitigation Strategies (REMS) to help, e.g., ensure that substantially no drug remains in the drug delivery device so that the drug deliverable therefrom is not accessed by an unauthorized party. A person skilled in the art will appreciate that the drug holder may not have precisely zero drug therein but nevertheless be considered to have substantially no drug therein due to any number of factors, such as sensitivity of measurement equipment.

The device indicator can have a variety of configurations. In this illustrated embodiment, the device indicator includes the elongate shafts 524, 526 of the plungers 512, 514. A first, distal portion of the first elongate shaft 524 is a first color, and a second, proximal portion of the first elongate shaft 524 is a second, different color. FIGS. 9 and 10 show the first color visible through each of the viewing windows 528, 530. The first color is white as illustrated, although another color can be used. The first color being visible in a viewing window 528, 530 can indicate that the dose associated with the viewing window 528, 530, e.g., the first dose for the first viewing window 528 and the second dose for the second viewing window 530, has not occurred. The second color being visible in a viewing window 528, 530 can indicate that the dose associated with the viewing window 528, 530 has occurred. When the plungers 512, 514 respectively move distally relative to the body 518 during drug delivery, the color visible through a viewing window 528, 530 can change. In other embodiments, instead of or in addition to different colors, another marking can be configured to be visible through the viewing windows 528, 530 to indicate whether the first and second doses have been dispensed, such as symbols (e.g., stars, triangles, "X"s, a thumbs up, a check mark, etc.), numbers (e.g., "1" for the first dose and "2" for the second dose), and/or letters (e.g., "A" for the first dose" and "B" for the second dose).

In an exemplary embodiment of using the drug delivery device 500, the drug delivery device 500 has an initial configuration, shown in FIGS. 9 and 10, in which the plungers 512, 514 are each in a first configuration. The first color is visible through each of the viewing windows 528, 530. As shown in FIG. 11, the first plunger 512 is moved distally, e.g., pushed by a user on the first proximal flange 520 as shown by arrow A1, relative to the body 518 and relative to the second plunger 514 to cause a first dose of the drug to be delivered. The user can hold the second plunger 514 with fingers on the proximal flange 522 thereof applying a proximal force thereto, as shown by arrows A2, which holds the second plunger 514 in position relative to the body 518 during the actuation of the first plunger 512. The second plunger 514 is configured to stop distal movement of the first plunger 512 after the first plunger 512 has moved a first distance D1 (see FIG. 9) that is defined by a length of the first plunger 512 that extends proximally beyond the second plunger 514. The second plunger 514 prevents the first plunger 512 from moving distally more than the distance D1 by including a cavity 532 in the second proximal flange 522 that is configured to seat the first proximal flange 520 therein such that the user cannot continue pushing the first plunger 512 distally. Instead of having the cavity 532, a distal surface of the first plunger 512, e.g., a distal surface of the first proximal flange 520, can be configured to abut a proximal surface of the second plunger 514, e.g., a proximal surface of the second proximal flange 522, such that the user cannot continue pushing the first plunger 512 distally.

FIG. 12 illustrates the drug delivery device 500 after the first dose has been delivered. The second color is now visible through the first viewing window 528 to indicate that the first dose has been delivered. The first color remains visible through the second viewing window 530 to indicate that the second dose has not been delivered.

After the first dose has been delivered, e.g., after the first stage of operation, the second plunger 514 is moved distally, e.g., pushed by a user on the second proximal flange 522 as shown by arrow A3, relative to the body 212 to cause a second dose of the drug to be delivered. The user can hold the body 518 with fingers on a proximal flange 534 thereof applying a proximal force thereto, as shown by arrows A2, which holds the second plunger 514 in position relative to the body 518 during the actuation of the first plunger 512. A distal surface of the second plunger 514, e.g., a distal surface of the second proximal flange 522, is configured to abut a proximal surface of the body 518, e.g., a proximal surface of the body's proximal flange 534, such that the user cannot continue pushing the second plunger 514 distally. FIG. 13 illustrates the drug delivery device 500 after the second dose has been delivered. The second color is visible through the second viewing window 530 to indicate that the second dose has been delivered. The second color remains visible through the first viewing window 528 to indicate that the first dose has been delivered.

The drug delivery device 500 in this illustrated embodiment is not powered, e.g., does not include any electrical components such as a processor, a sensor, a memory, a communications interface, etc. The drug delivery device 500 thus does not need to include a power supply though a power supply could be included, e.g., for connectivity, for powering a light source of the drug delivery device, etc.

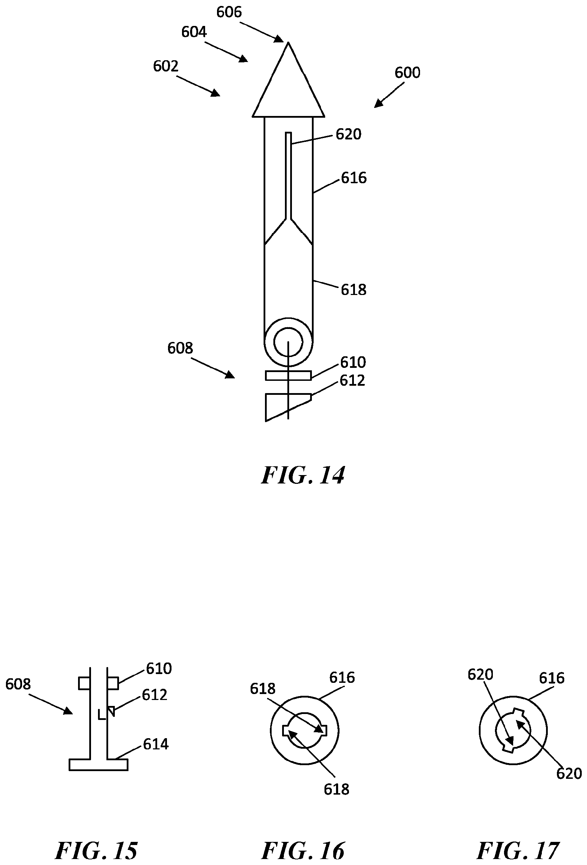

FIG. 14 illustrates another exemplary embodiment of the drug delivery device 100 of FIG. 1. FIG. 14 illustrates a drug delivery device 600 configured to expel a drug into a nose of a patient in two stages of operation. In a first stage of operation a first dose of the drug is delivered, and in a second stage of operation a second dose of the drug is delivered. The drug delivery device 600 is generally configured and used similar to that discussed above regarding FIG. 1 and includes a dispensing head 602 that includes a tip 604 and an opening 606. The tip 604 is configured to inserted into a first nostril during a first stage of operation of the drug delivery device 600 and into a second nostril during a second stage of operation of the drug delivery device 600. The drug in this illustrated embodiment is configured to be self-administered such that the user who actuates the drug delivery device 600 is the person receiving the drug from the drug delivery device 600, although another person can actuate the device 600 for delivery into another person.

In this illustrated embodiment, an actuator of the drug delivery device 600 includes a plunger 608 that is configured to be pushed distally toward the dispensing head 602 to cause the drug to be delivered from the drug delivery device 600. The plunger 608 is operatively engaged with a stopper located proximal to a drug in the drug delivery device's drug holder and pushes drug out of the drug holder similar to that discussed above regarding the stopper 312. As also shown in FIG. 15, the plunger 608 includes first frangible tabs 610 and second frangible tabs 612. The plunger 608 includes two first frangible tabs 610 and two second frangible tabs 612 in this illustrated embodiment but can include another number, e.g., three, four, five, six, etc. The frangible tabs 610, 612 can be made from any of a variety of materials configured to allow breakage of the frangible tabs 610, 612, such as a polymer or a metal. The frangible tabs 610, 612 have a sufficiently small thickness to allow for their breakage. The thickness will vary based on the material of the frangible tabs 610, 612.

In an exemplary embodiment of using the drug delivery device 600, the drug delivery device 600 has an initial configuration, shown in FIG. 14, in which all of the frangible tabs 610, 612 of the plunger 608 are intact, e.g., not broken. The plunger 608 is moved distally, e.g., pushed by a user on a proximal flange 614 of the plunger 608, relative to the drug delivery device's body 616 to cause a first dose of the drug to be delivered.

The body 616 of the drug delivery device 600 includes first, proximal grooves 618 and second, distal grooves 620 therein. As also shown in FIGS. 16 and 17, the first and second grooves 618, 620 are located about ninety degrees apart from one another. The body 616 in FIG. 17 is rotated about ninety degrees from the body 616 in FIG. 16. In the first stage of operation, the distal movement of the plunger 608 causes the first frangible tabs 610 to break due to the force being applied to the plunger 608. The plunger 608 moves distally in the first stage of operation, but the first frangible tabs 610 break instead of moving distally with a remainder of the plunger 608 due to the first frangible tabs 610, e.g., a distal surface thereof, abutting the body 616, e.g., a proximal surface thereof, before the first actuation of the plunger 608. The breakage of the first frangible tabs 610 helps provide a high-speed actuation that facilitates production of a fine mist of drug to be delivered from the opening 606. A proximal stop surface of the first grooves 618 is configured to stop distal movement of the plunger 608 by the second tabs 612, e.g., distal surfaces thereof, abutting the proximal stop surface of the first grooves 618 at the end of the first stage of operation.

After the first dose has been delivered, e.g., after the first stage of operation, the plunger 608 is rotated about ninety degrees. The plunger 608 is moved further distally relative to the drug delivery device's body 616 to cause a second dose of the drug to be delivered. The further distal movement of the plunger 608 causes the second frangible tabs 612 to break due to the force being applied to the plunger 608. The breakage of the second frangible tabs 612 helps provide a high-speed actuation that facilitates production of a fine mist of drug to be delivered from the opening 606. The plunger 608 moves distally in the second stage of operation, but the second frangible tabs 612 break instead of moving distally with a remainder of the plunger 608 due to the second frangible tabs 612, e.g., a distal surface thereof, abutting the end of the first grooves 618 before the second actuation of the plunger 608.

The drug delivery device 600 in this illustrated embodiment is not powered, e.g., does not include any electrical components such as a processor, a sensor, a memory, a communications interface, etc. The drug delivery device 600 thus does not need to include a power supply though a power supply could be included, e.g., for connectivity, for powering a light source of the drug delivery device, etc.

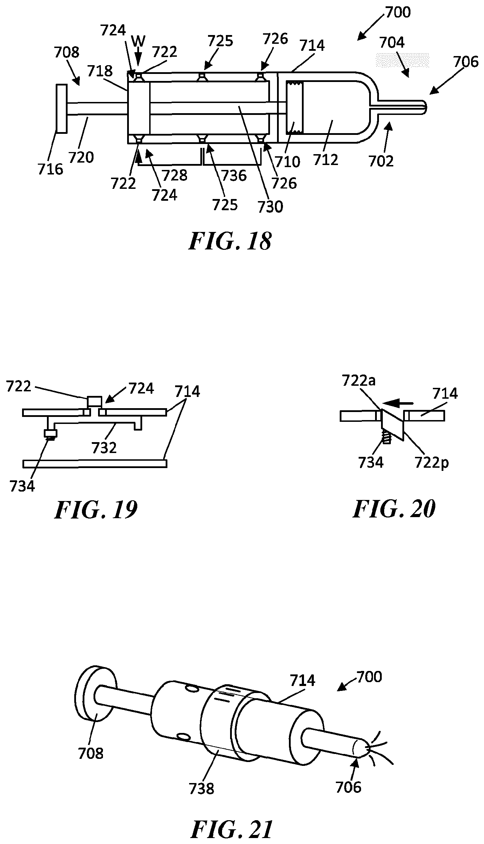

FIG. 18 illustrates another exemplary embodiment of the drug delivery device 100 of FIG. 1. FIG. 18 illustrates a drug delivery device 700 configured to expel a drug into a nose of a patient in two stages of operation. In a first stage of operation a first dose of the drug is delivered, and in a second stage of operation a second dose of the drug is delivered. The drug delivery device 700 is generally configured and used similar to that discussed above regarding FIG. 1 and includes a dispensing head 702 that includes a tip 704 and an opening 706. The tip 704 is configured to inserted into a first nostril during a first stage of operation of the drug delivery device 700 and into a second nostril during a second stage of operation of the drug delivery device 700. The drug in this illustrated embodiment is configured to be self-administered such that the user who actuates the drug delivery device 700 is the person receiving the drug from the drug delivery device 700, although another person can actuate the device 700 for delivery into another person.

In this illustrated embodiment, an actuator of the drug delivery device 700 includes a plunger 708 that is configured to be pushed distally toward the dispensing head 702 to cause the drug to be delivered from the drug delivery device 700. The plunger 708 in this illustrated embodiment is spring-loaded. The plunger 708 is operatively engaged with a stopper 710 located proximal to a drug in the drug delivery device's drug holder 712 and pushes drug out of the drug holder similar to that discussed above regarding the stopper 312.

As also shown in FIG. 19, the plunger 708 includes a proximal flange 716, a distal flange 718, and an elongate shaft 720 extending between the proximal and distal flanges 716, 718. The proximal flange 716 is configured to be pushed by a user to actuate the drug delivery device 700. The distal flange 718 includes a plurality of depressible pins 722 extending radially outward therefrom. The distal flange 718 includes two pins 722 in this illustrated embodiment but can include another number of pins 722, e.g., one, three, four, five, six, etc. In an exemplary embodiment, the pins 722 are equidistantly spaced around a circumference of the distal flange 718, as with the drug delivery device 700. The pins 722 being equidistantly spaced around the circumference of the distal flange 718 may help provide for smooth, level movement of the distal flange 718 within a body 714 of the drug delivery device 700.

The body 714 includes a plurality of proximal holes 724 formed in a sidewall thereof, a plurality of intermediate holes 725 formed in a sidewall thereof, and a plurality of distal holes 726 formed in the sidewall thereof. The proximal holes 724, the intermediate holes 725, and the distal holes 726 are configured to seat the pins 722 therein. At various times, as discussed further below, the drug delivery device 700 is configured such that all of the proximal holes 724 seat all of the pins 722 therein, all of the intermediate holes 725 seat all of the pins 722 therein, all of the distal holes 726 seat all of the pins 722 therein, or none of proximal, intermediate, and distal holes 724, 725, 726 seat any of the pins 722 therein. A number of the proximal holes 724 equals a number of the pins 722, a number of the intermediate holes 725 equals a number of the pins 722, and a number of the distal holes 726 equals a number of the pins 722. In other embodiments, the body 714 can include more proximal holes 724, intermediate holes 725, and/or more distal holes 726 than a number of the pins 722, which may facilitate reuse of the body 714 with different plungers having different numbers of depressible pins.

In an exemplary embodiment of using the drug delivery device 700, the drug delivery device 700 has an initial configuration, shown in FIGS. 18 and 19, in which all of the pins 722 are seated in the proximal holes 724. The plunger 708 is actuated by depressing the pins 722 such that the pins 722 move radially inward relative to the body 714, as shown by arrow W in FIG. 18, to no longer be seated in the proximal holes 722. The pins 722 are depressed by being manually pressed by a user, e.g., by fingers/thumb of the user. The release of the pins 722 from the proximal holes 724 allows the plunger 708 to move a first distance 728 distally relative to the drug delivery device's body 714 to cause a first dose of the drug to be delivered. The first distance 728 is defined by a distance between the proximal holes 724 and the intermediate holes 725. The plunger 708 automatically moves distally when the pins 722 are pushed inward of the proximal holes 724 due to a bias spring 730 of the drug delivery device 700 that biases the plunger 708 distally.

The pins 722 are each biased radially outward by a spring base 732 from which the pin 722 extends radially outward, as shown in FIG. 19. Each of the spring bases 732 is operatively coupled to a second bias spring 734 that provides the radially outward bias. An interior surface 736 of the body 714 prevents the second bias springs 734 from urging the spring bases 732, and hence the pins 722, radially outward as the plunger 708 moves distally along the first distance 728. When the plunger 708 has moved distally enough for the pins 722 to align with the distal holes 726, e.g., when the plunger 708 has moved the first distance 728, the second bias springs 734 urge the spring bases 732, and hence the pins 722, radially outward such that the pins 722 become seated in the intermediate holes 725. The seating of the pins 722 in the intermediate holes 725 causes the plunger 708 to stop moving distally and thereby ends the first stage of operation.

After the first dose has been delivered, e.g., after the first stage of operation, the plunger 708 is actuated again by depressing the pins 722 such that the pins 722 move radially inward relative to the body 714 to no longer be seated in the intermediate holes 725. The plunger 708 thus moves distally under force of the bias spring 730, as discussed above with respect to the first stage of operation, to cause a second dose of the drug to be delivered. When the plunger 708 has moved distally enough for the pins 722 to align with the distal holes 726, e.g., when the plunger 708 has moved a second distance 736, the second bias springs 734 urge the spring bases 732, and hence the pins 722, radially outward such that the pins 722 become seated in the distal holes 726. The second distance 736 is defined by a distance between the intermediate holes 725 and the distal holes 726. The seating of the pins 722 in the distal holes 726 causes the plunger 708 to stop moving distally and thereby ends the second stage of operation.

The pins 722 in this illustrated embodiment have a rectangular shape, as shown in FIG. 19. The pins 722 can, however, have another shape, such as bullet-shaped, triangular, etc. FIG. 20 illustrates another embodiment of the pins 722 in which pins 722a each have a sloped outer surface that slopes radially inward in a distal-to-proximal direction. The sloped outer surface may facilitate the pins 722a "popping" back into holes formed in the body 714, e.g., into either the intermediate holes 725 or the distal holes 726, after having been previously pressed radially inward.

FIG. 20 also illustrates an embodiment in which the pins 722 are not spring-biased using the spring base 732. Instead, the pins 722a are each pivotally coupled to the body 714 at a pivot point 722p and directly engaged by the second bias spring 734.

**In** the exemplary method discussed above, the pins 722 are manually pressed by a user's hand to actuate the plunger 708. Alternatively, an accessory can be used to actuate the plunger 708 by depressing the pins 722. Some users may lack sufficient dexterity and/or strength to depress the pins 722 at all and/or simultaneously. The accessory may facilitate pin 722 depression at least for these users since pin 722 depression is not reliant on user dexterity or strength.

FIG. 21 illustrates one embodiment of an accessory 738 configured to depress the pins 722. The accessory 738 in this illustrated embodiment includes a ring that circumscribes the body 714. The accessory 738 can be pre-loaded on the body 714, which may help ensure that the pins 722 are not prematurely depressed to move out of the proximal holes 724. FIG. 21 illustrates the accessory 738 in an exemplary initial, pre-loaded position in which the accessory 738 is positioned over the intermediate holes 725. Alternatively, the accessory 738 can be loaded on the body 714 by a user by sliding the accessory 738 over the body 714 in a proximal direction until the accessory 738 is positioned around the body 714 at a location distal to the proximal holes 724.

In an exemplary embodiment of using the drug delivery device 700 and the accessory 738, with the drug delivery device 700 in the initial configuration, the plunger 708 is actuated by moving the accessory 738, e.g., sliding the accessory 738 along the body 714, to be positioned over the proximal holes 724, which cause the pins 722 to move radially inward and the plunger 708 to move distally until the pins 722 engage the intermediate holes 725. The accessory 738 is then moved again to be positioned over the intermediate holes 725, which cause the pins 722 to move radially inward and the plunger 708 to move distally until the pins 722 engage the distal holes 726.

The drug delivery device 700 in this illustrated embodiment is not powered, e.g., does not include any electrical components such as a processor, a sensor, a memory, a communications interface, etc. The drug delivery device 700 thus does not need to include a power supply though a power supply could be included, e.g., for connectivity, for powering a light source of the drug delivery device, etc.

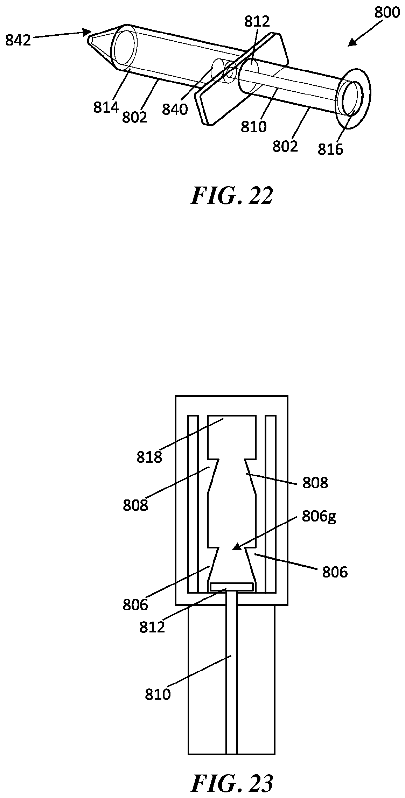

FIG. 22 illustrates another exemplary embodiment of the drug delivery device 100 of FIG. 1. FIG. 22 illustrates a drug delivery device 800 configured to expel a drug into a nose of a patient in two stages of operation. In a first stage of operation a first dose of the drug is delivered, and in a second stage of operation a second dose of the drug is delivered. The drug delivery device 800 is generally configured and used similar to that discussed above regarding FIG. 1 except that the drug delivery device 800 includes a housing 802 configured to facilitate delivery of two drug doses from the drug delivery device 800. The drug delivery device's tip 804 is configured to inserted into a first nostril during a first stage of operation of the drug delivery device 800 and into a second nostril during a second stage of operation of the drug delivery device 800. The drug in this illustrated embodiment is configured to be self-administered such that the user who actuates the drug delivery device 800 is the person receiving the drug from the drug delivery device 800, although another person can actuate the device 800 for delivery into another person.

As shown in FIG. 23, the housing 802 includes a first, proximal internal stop member 806 and a second, distal internal stop member 808. The first and second internal stop members 806, 808 are configured to cooperate with the drug delivery device's plunger 810 to facilitate delivery of the two drug doses. The first and second internal stop members 806, 808 extend circumferentially around the housing 802 in this illustrated embodiment, which allows each of the stop members 806, 808 to continuously engage around a surface of the plunger's distal flange 812 to help hold the plunger 810 in position relative to the drug delivery device's body 814, as discussed further below. However, one or both of the first and second internal stop members 806, 808 can extend around only a partial circumference of the housing 802.

In an exemplary embodiment of using the drug delivery device 800, the drug delivery device 800 has an initial configuration, shown in FIGS. 22 and 23, in which the plunger 810, e.g., the distal flange 812 thereof, is engaged with the first internal stop member 806. The engagement of the plunger 810 with the first internal stop member 806 holds the plunger 810 in a fixed position relative to the body 814. The plunger 810 is moved distally, e.g., pushed by a user on a proximal flange 816 of the plunger 810, relative to the housing 802 and relative to the drug delivery device's body 814 to cause a first dose of the drug to be delivered. The distal movement of the plunger 810 causes the plunger's distal flange 812 to move distally past the first internal stop member 806. The first internal stop member 806 can, as in this illustrated embodiment, have a sloped surface that slopes radially inward in a proximal-to-distal direction, which may facilitate the plunger's distal movement past the first internal stop member 806 by providing a sloped guide surface along which the distal flange 812 can slide. The plunger 810 overcoming the first internal stop member 806 distally may help push the drug delivery device's stopper 840 at a higher speed, which may cause the drug to be sprayed in a finer mist out of the drug delivery device's opening 842. The second internal stop member 808 is configured to stop the distal movement of the plunger 810 by engaging with the plunger 810, e.g., by a proximal surface of the second internal stop member 808 engaging a distal surface of the distal flange 812, and thus end the first stage of operation.

The first internal stop member 806 is configured to prevent the plunger 810 from being pulled proximally past the first internal stop member 806 after the plunger 810 has moved distally past the first internal surface member 806 by defining a distal opening 806g that has a diameter that is less than a diameter of the distal flange 812. The first internal stop member 806 can be a resilient member configured to flex inwardly when a sufficient force is applied thereto. In this way, when the sufficient force (or greater) is applied to the first internal stop member 806 by the plunger 810, e.g., the distal flange 812, pushing thereagainst in a distal direction, the first internal stop member 806 can flex radially inward to facilitate movement of the plunger's distal flange 812 distally past the first internal stop member 806. The force sufficient to cause the first internal stop member 806 to flex will vary based on a thickness of and on a material, e.g., a polymer or a metal, of the first internal stop member 806 and the distal flange 812.

After the first dose has been delivered, e.g., after the first stage of operation, the plunger 810 is moved further distally relative to the housing 802 and the body 814 to cause a second dose of the drug to be delivered. The further distal movement of the plunger 810 causes the distal flange 812 to become disengaged from the second internal stop member 808, similar to that discussed above regarding the distal flange 812 becoming disengaged from the first internal stop member 806, to cause a second dose of the drug to be delivered. The plunger 810 overcoming the second internal stop member 808 distally may help push the drug delivery device's stopper 840 at a higher speed, which may cause the drug to be sprayed in a finer mist out of the drug delivery device's opening 842. The second internal stop member 808 can, as in this illustrated embodiment, have a sloped surface that slopes radially inward in a proximal-to-distal direction, which may facilitate the plunger's distal movement past the second internal stop member 808 by providing a sloped guide surface along which the distal flange 812 can slide. An internal stop surface 818 of the housing 802 is configured to stop the distal movement of the plunger 810 by engaging with the plunger 810, e.g., by the internal stop surface 818 engaging the distal surface of the distal flange 812, and thus end the second stage of operation.

The second internal stop member 808 is configured to prevent the plunger 810 from being pulled proximally past the second internal stop member 808 after the plunger 810 has moved distally past the second internal surface member 808 by defining a distal opening 808g that has a diameter that is less than the diameter of the distal flange 812. The second internal stop member 808 can be a resilient member configured to flex inwardly when a sufficient force is applied thereto similar to that discussed above regarding the first internal stop member 806.

The drug delivery device 800 in this illustrated embodiment is not powered, e.g., does not include any electrical components such as a processor, a sensor, a memory, a communications interface, etc. The drug delivery device 800 thus does not need to include a power supply though a power supply could be included, e.g., for connectivity, for powering a light source of the drug delivery device, etc.

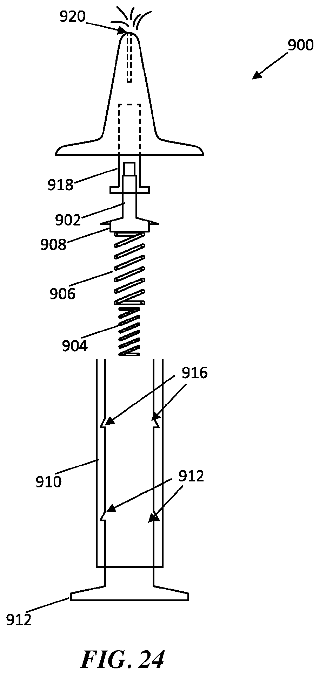

FIG. 24 illustrates another exemplary embodiment of the drug delivery device 100 of FIG. 1. FIG. 24 illustrates a drug delivery device 900 configured to expel a drug into a nose of a patient in two stages of operation. In a first stage of operation a first dose of the drug is delivered, and in a second stage of operation a second dose of the drug is delivered. The drug delivery device 900 is generally configured and used similar to that discussed above regarding the drug delivery device 700 of FIG. 18 except that the drug delivery device's plunger 902 is spring-loaded using a first bias spring 904 and a second bias spring 906 instead of the one bias spring 730.

In an exemplary embodiment of using the drug delivery device 900, the drug delivery device 900 has an initial configuration in which a distal flange 908 of the plunger 902 is seated in a first, proximal groove 912 formed in an internal surface of the drug delivery device's body 910. The first groove 912, as well as a second, distal groove 916 formed in the internal surface of the drug delivery device's body 910, extends circumferentially around the body 912 in this illustrated embodiment, which allows each of the grooves 912, 916 to continuously engage around a surface of the plunger's distal flange 908 to help hold the plunger 902 in position relative to the drug delivery device's body 910, as discussed further below. However, one or both of the first and second grooves 912, 916 can extend around only a partial circumference of the body 910.

The drug delivery device 900 is first actuated by actuating a trigger 914 of the drug delivery device 900. The trigger 914 is operatively coupled to the plunger 902, e.g., by a distal surface of the trigger 914 abutting a proximal surface of the plunger 902. Actuating the trigger 914 includes pushing the trigger 914 distally, which causes the plunger 902 to be pushed distally. The distal movement of the plunger 902 causes the plunger 902 to exit the first groove 912, which allows the first bias spring 904 to urge the plunger 902 a first distance distally relative to the drug delivery device's body 910 to cause a first dose of drug from the drug delivery device's drug holder 918 to be delivered out of the drug delivery device's opening 920. The first distance is defined by a distance between the proximal groove 912 and the distal groove 916. The plunger 902 automatically moves distally when the plunger 902 exits the first groove 912 due to the first bias spring 904 biasing the plunger 902 distally. When the plunger 902 has moved distally enough for the distal flange 908 to align with the second groove 916, e.g., when the plunger 902 has moved the first distance, the distal flange 908 becomes seated in the second groove 916. The seating of the distal flange 908 in the second groove 916 causes the plunger 902 to stop moving distally and thereby ends the first stage of operation. The distal flange 908 can include one or more pins similar to the pins 722 of FIG. 18 discussed above to facilitate the seating of the distal flange 908 in and disengaging of the distal flange 908 from the first groove 912 and the second groove 916.

After the first dose has been delivered, e.g., after the first stage of operation, the trigger 914 is actuated again such that the plunger 902 moves distally relative to the body 910 under force of the second bias spring 906 so the distal flange 908 is no longer be seated in the second groove 916. An internal stop surface of the housing body 910 is configured to stop the distal movement of the plunger 902 by engaging with the plunger 902, e.g., by the internal stop surface engaging a distal surface of the distal flange 908, and thus end the second stage of operation.

The drug delivery device 900 in this illustrated embodiment is not powered, e.g., does not include any electrical components such as a processor, a sensor, a memory, a communications interface, etc. The drug delivery device 900 thus does not need to include a power supply though a power supply could be included, e.g., for connectivity, for powering a light source of the drug delivery device, etc.

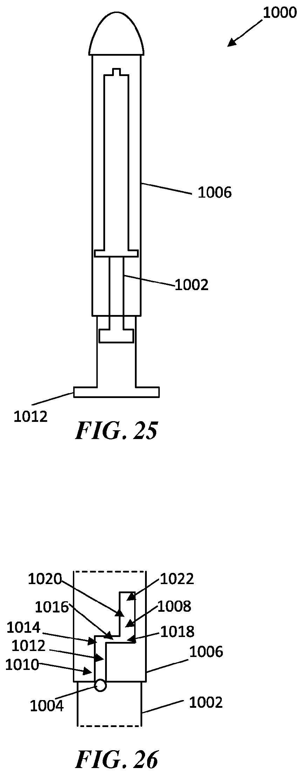

FIG. 25 illustrates another exemplary embodiment of the drug delivery device 100 of FIG. 1. FIG. 25 illustrates a drug delivery device 1000 configured to expel a drug into a nose of a patient in two stages of operation. In a first stage of operation a first dose of the drug is delivered, and in a second stage of operation a second dose of the drug is delivered. The drug delivery device 1000 is generally configured and used similar to that discussed above regarding FIG. 1 except that, as also shown in FIG. 26, a plunger 1002 of the drug delivery device 1000 includes a cam 1004 and a body 1006 of the drug delivery device 1000 includes a track 1008 in which the cam 1004 is slidably movable.

In an exemplary embodiment of using the drug delivery device 1000, the drug delivery device 1000 has an initial configuration, shown in FIG. 26, in which the cam 1004 is positioned just distal to an open distal end 1010 of the track 1008. The plunger 1002 is moved distally, e.g., by actuating a trigger 1012 of the drug delivery device 1000 that is operatively engaged with the plunger 1002 similar to the trigger 912 of FIG. 24, relative to the drug delivery device's body 1006 to cause a first dose of the drug to be delivered. The cam 1004 enters into and slides in a first longitudinal or vertical portion 1012 of the track 1008 during the first stage of operation. A proximal end 1014 of the first longitudinal portion 1012 of the track 1008 is configured to stop the proximal movement of the plunger 1002 by the cam 1004 abutting the proximal end 1014 of the first longitudinal portion 1012 of the track 1008. In other embodiments, the distal end of the track 1008 is closed instead of being open, and with the drug delivery device 1000 in the initial configuration the cam 1004 is positioned within the track 1008 at the track's closed distal end.

After the first dose has been delivered, e.g., after the first stage of operation, the plunger 1002 is rotated relative to the body 1006, e.g., by rotating the trigger 1012. The rotation of the plunger 1002 causes the cam 1004 to slide within an axial or horizontal portion 1016 of the track 1008. The plunger 1002 cannot be rotated relative to the body 1006 during the first stage of operation due to the cam's location within the first longitudinal portion 1012 of the track 1008. In embodiments in which, in the initial configuration, the cam 1004 is positioned within the track 1008 at the track's closed distal end, the plunger 1002 is prevented from rotating relative to the body 1006 before the first stage of operation due to the cam's location within the first longitudinal portion 1012 of the track 1008. An end 1018 of the axial portion 1016 of the track 1008 is configured to stop the rotational movement of the plunger 1002 by the cam 1004 abutting the end 1018.

After the first dose has been delivered, e.g., after the first stage of operation, the plunger 1002 is moved further distally relative to the drug delivery device's body 1006, e.g., by actuating the trigger 1012 again, to cause a second dose of the drug to be delivered. The cam 1004 slides in a second longitudinal or vertical portion 1020 of the track 1008 during the second stage of operation. A proximal end 1022 of the second longitudinal portion 1020 of the track 1008 is configured to stop the proximal movement of the drug holder 1002 by the cam 1004 abutting the proximal end 1022 of the second longitudinal portion 1020 of the track 1008 and thus end the second stage of operation.

In other embodiments, the track 1008 can include at least one additional longitudinal or vertical portion and at least one additional axial or horizontal portion with each additional vertical portion and horizontal portion pair allowing for one additional drug delivery.

The drug delivery device 1000 in this illustrated embodiment is not powered, e.g., does not include any electrical components such as a processor, a sensor, a memory, a communications interface, etc. The drug delivery device 1000 thus does not need to include a power supply though a power supply could be included, e.g., for connectivity, for powering a light source of the drug delivery device, etc.

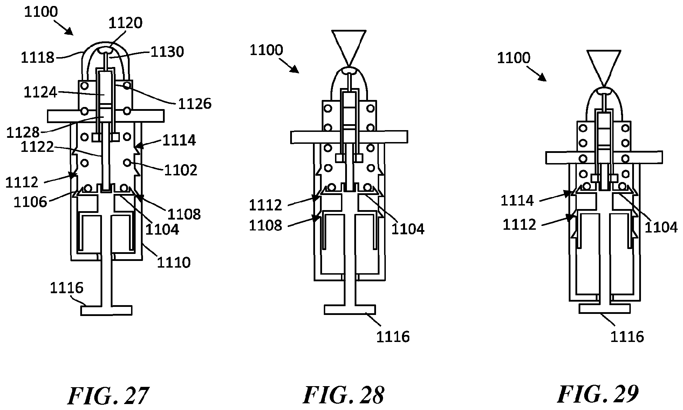

FIG. 27 illustrates another exemplary embodiment of the drug delivery device 100 of FIG. 1. FIG. 27 illustrates a drug delivery device 1100 configured to expel a drug into a nose of a patient in two stages of operation. In a first stage of operation a first dose of the drug is delivered, and in a second stage of operation a second dose of the drug is delivered. The drug delivery device 1100 is generally configured and used similar to that discussed above regarding the drug delivery device 900 of FIG. 24 except that the drug delivery device 1100 includes one bias spring 1102 and a flexible clip 1104 instead of the two bias springs 904, 906.

In an exemplary embodiment of using the drug delivery device 1100, the drug delivery device 900 has an initial configuration, shown in FIG. 27, in which a flange 1106 of the flexible clip 1104 is seated in a first groove 1108 formed in an internal surface of the drug delivery device's body 1110. The first groove 1108, as well as second and third grooves 1112, 1114 formed in the internal surface of the drug delivery device's body 1110, extends circumferentially around the body 1110 in this illustrated embodiment, which allows each of the grooves 1108, 1112, 1114 to continuously engage around a surface of the flange 1106 to help hold the drug delivery device's plunger 1116 and plunger rod 1122 in position relative to the drug delivery device's body 1110, as discussed further below. However, one or more of the grooves 1108, 1112, 1114 can extend around only a partial circumference of the body 1106.

In this illustrated embodiment, a protective cap 1118 is positioned over the drug delivery device's opening 1120. The protective cap 1118 is configured to be removed before actuation of the drug delivery device 1110. Any of the embodiments of drug delivery devices described herein can similarly include a protective cap.

After removal of the protective cap 1118, the drug delivery device 1100 is first actuated by actuating the plunger 1116. Actuating the plunger 1116 includes pushing the plunger 1116 distally, which causes the plunger 1116 to be pushed distally. The distal movement of the plunger 1116 causes the flexible clip 1104 to flex such that the flange 1106 exits the first groove 1108, which allows the bias spring 1102 to urge the plunger 1116, the flexible clip 1104, and the plunger rod 1122 a first distance distally relative to the drug delivery device's body 1110 to cause a first dose of drug 1124 from the drug delivery device's drug holder 1126 to be delivered out of the opening 1120. The plunger rod 1122 is operatively engaged with a stopper 1128 in the drug holder 1126, so the movement of the plunger rod 1122 causes the stopper 1128 to move and urge the drug 1124 out of the drug holder 1126, through a cannula 1130, and out the opening 1120. The first distance is defined by a distance between the first groove 1108 and the second groove 1112. The plunger 1116, the flexible clip 1104, and the plunger rod 1122 automatically move distally when the flange 1106 exits the first groove 1108 due to the bias spring 1102 providing a distal bias. As shown in FIG. 28, when the flexible clip 1104 has moved distally enough for the flange 1106 to align with the second groove 1112, e.g., when the plunger 1116, the flexible clip 1104, and the plunger rod 1122 have moved the first distance, the flange 1106 becomes seated in the second groove 1112. The seating of the flange 1106 in the second groove 1112 causes the plunger 1116, the flexible clip 1104, and the plunger rod 1122 to stop moving distally and thereby ends the first stage of operation.

After the first dose has been delivered, e.g., after the first stage of operation, the plunger 1116 is actuated again such that the plunger 1116, the flexible clip 1104, and the plunger rod 1122 move distally relative to the body 1110. The distal movement of the plunger 1116 causes the flexible clip 1104 to again flex such that the flange 1106 exits the second groove 1112, which allows the bias spring 1102 to urge the plunger 1116, the flexible clip 1104, and the plunger rod 1122 a second distance distally relative to the drug delivery device's body 1110 to cause a second dose of the drug 1124 from the drug delivery device's drug holder 1126 to be delivered out of the opening 1120. As shown in FIG. 29, when the flexible clip 1104 has moved distally enough for the flange 1106 to align with the third groove 1114, e.g., when the plunger 1116, the flexible clip 1104, and the plunger rod 1122 have moved the second distance, the flange 1106 becomes seated in the third groove 1114. The seating of the flange 1106 in the third groove 1114 causes the plunger 1116, the flexible clip 1104, and the plunger rod 1122 to stop moving distally and thereby ends the second stage of operation.

The drug delivery device 1100 in this illustrated embodiment is not powered, e.g., does not include any electrical components such as a processor, a sensor, a memory, a communications interface, etc. The drug delivery device 1100 thus does not need to include a power supply though a power supply could be included, e.g., for connectivity, for powering a light source of the drug delivery device, etc.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. In particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that modifications within the scope of the claims may be made without departing from the overall scope of the present disclosure.

## Claims

1. A drug delivery system (700), comprising:
a dispensing head (702) including a proximal tip (704) configured to be positioned in a nose of a patient, the tip having an opening (706) therein,
a drug holder (712) containing a drug therein;
a plunger (708) configured to move distally in response to a manual user force applied thereto;
a body (714) in which the drug holder is disposed;
and
a mechanical stop;
wherein the plunger is configured to move a first predetermined distance distally relative to the dispensing head in a first stage of operation in which the drug is delivered from the drug holder and out of the opening before the mechanical stop prevents further distal movement of the plunger; and
wherein the plunger is configured to move a second predetermined distance distally relative to the dispensing head in a second stage of operation in which the drug is delivered from the drug holder and out of the opening;
and **characterized in that** the body has a proximal hole (724) formed in and extending through a sidewall thereof, a distal hole (726) formed in and extending through the sidewall, and an intermediate hole (736) formed in and extending through the sidewall, the intermediate hole being located between the proximal and distal holes; and
the mechanical stop comprises a depressible pin (722) that extends radially outward from the plunger and that is configured to be successively seated in and extend through the proximal hole, the intermediate hole, and the distal hole.

2. The system of claim 1, wherein the depressible pin is configured to be manually pushed radially inward relative to the body after the plunger moves the first predetermined distance and before the plunger moves the second predetermined distance.

3. The system of any preceding claim, further comprising a viewing window (528) configured to allow a user to visualize whether or not each of the first and second stages of operation have occurred.

4. The system of any preceding claim, wherein the drug is one of ketamine, esketamine, naloxone, and sumatriptan.

## Patentansprüche

1. Arzneimittelabgabesystem (700), umfassend:
einen Ausgabekopf (702), der eine proximale Spitze (704) einschließt, die konfiguriert ist, um in einer Nase eines Patienten positioniert zu werden, wobei die Spitze eine Öffnung (706) darin aufweist,
einen Arzneimittelhalter (712), der ein Arzneimittel darin enthält;
einen Kolben (708), der konfiguriert ist, um sich als Reaktion auf eine darauf ausgeübte manuelle Benutzerkraft distal zu bewegen;
ein Gehäuse (714), in dem der Arzneimittelhalter angeordnet ist;
und
einen mechanischen Anschlag;
wobei der Kolben konfiguriert ist, um sich in einer ersten Betriebsphase, in der das Arzneimittel von dem Arzneimittelhalter und aus der Öffnung abgegeben wird, bevor der mechanische Anschlag eine weitere distale Bewegung des Kolbens verhindert, eine erste vorbestimmte Distanz distal relativ zu dem Ausgabekopf zu bewegen; und
wobei der Kolben konfiguriert ist, um sich in einer zweiten Betriebsphase, in der das Arzneimittel von dem Arzneimittelhalter und aus der Öffnung abgegeben wird, eine zweite vorbestimmte Distanz distal relativ zu dem Ausgabekopf zu bewegen;
und **dadurch gekennzeichnet, dass** das Gehäuse ein proximales Loch (724), das in einer Seitenwand davon ausgebildet ist und sich durch diese hindurch erstreckt, ein distales Loch (726), das in der Seitenwand ausgebildet ist und sich durch diese hindurch erstreckt, und ein intermediäres Loch (736), das in der Seitenwand ausgebildet ist und sich durch diese hindurch erstreckt, aufweist, wobei sich das intermediäre Loch zwischen dem proximalen und dem distalen Loch befindet; und
der mechanische Anschlag einen niederdrückbaren Stift (722), der sich radial nach außen von dem Kolben erstreckt und der konfiguriert ist, um sukzessive in dem proximalen Loch, dem intermediären Loch und dem distalen Loch aufgenommen zu werden und sich durch diese zu erstrecken, umfasst.

2. System nach Anspruch 1, wobei der niederdrückbare Stift konfiguriert ist, um manuell radial nach innen relativ zu dem Gehäuse gedrückt zu werden, nachdem sich der Kolben die erste vorbestimmte Distanz bewegt hat und bevor sich der Kolben die zweite vorbestimmte Distanz bewegt.

3. System nach einem der vorstehenden Ansprüche, ferner umfassend ein Sichtfenster (528), das konfiguriert ist, um es einem Benutzer zu ermöglichen, zu sehen, ob die erste und die zweite Betriebsphase jeweils stattgefunden haben oder nicht.

4. System nach einem der vorstehenden Ansprüche, wobei das Arzneimittel eines von Ketamin, Esketamin, Naloxon oder Sumatriptan ist.

## Revendications

1. Système d'administration de médicament (700), comprenant :
une tête de distribution (702) comportant un embout proximal (704) conçu pour être positionné dans un nez d'un patient, l'embout ayant une ouverture (706) à l'intérieur de celui-ci,
un réservoir de médicament (712) contenant un médicament à l'intérieur de celui-ci ;
un piston (708) conçu pour se déplacer distalement en réponse à une force manuelle de l'utilisateur appliquée à celui-ci ;
un corps (714) dans lequel le réservoir de médicament est disposé ;
et
une butée mécanique ;
dans lequel le piston est conçu pour se déplacer sur une première distance prédéterminée distalement par rapport à la tête de distribution lors d'une première étape de fonctionnement au cours de laquelle le médicament est délivré depuis le réservoir de médicament et hors de l'ouverture avant que la butée mécanique n'empêche tout autre mouvement distal du piston ; et
dans lequel le piston est conçu pour se déplacer sur une seconde distance prédéterminée distalement par rapport à la tête de distribution lors d'une seconde étape de fonctionnement au cours de laquelle le médicament est distribué depuis le réservoir de médicament et hors de l'ouverture ;
et **caractérisé en ce que** le corps a un trou proximal (724) formé dans et s'étendant à travers une paroi latérale de celui-ci, un trou distal (726) formé dans et s'étendant à travers la paroi latérale, et un trou intermédiaire (736) formé dans et s'étendant à travers la paroi latérale, le trou intermédiaire étant situé entre les trous proximal et distal ; et
la butée mécanique comprend une goupille dépressible (722) qui s'étend radialement vers l'extérieur depuis le piston et qui est conçue pour être successivement logée dans et s'étendre à travers le trou proximal, le trou intermédiaire et le trou distal.

2. Système selon la revendication 1, dans lequel la goupille dépressible est conçue pour être poussée manuellement radialement vers l'intérieur par rapport au corps après que le piston s'est déplacé sur la première distance prédéterminée et avant que le piston ne se déplace sur la seconde distance prédéterminée.

3. Système selon l'une quelconque revendication précédente, comprenant en outre une fenêtre de visualisation (528) conçue pour permettre à un utilisateur de visualiser si chacune des première et seconde étapes de fonctionnement s'est produite ou non.

4. Système selon l'une quelconque revendication précédente, dans lequel le médicament est l'un parmi kétamine, eskétamine, naloxone et sumatriptan.
